(19)

**Europäisches Patentamt**
**European Patent Office**
**Office européen des brevets**

(11) **EP 4 336 186 A1**

(12) **EUROPEAN PATENT APPLICATION**

(43) Date of publication:
**13.03.2024   Bulletin 2024/11**

(21) Application number: **22195149.4**

(22) Date of filing: **12.09.2022**

(51) International Patent Classification (IPC):
**G01N 33/68** (2006.01)    **G01N 33/569** (2006.01)

(52) Cooperative Patent Classification (CPC):
**G01N 33/6893; G01N 33/56983;** G01N 2333/165;
G01N 2333/495; G01N 2333/70596; G01N 2800/12;
G01N 2800/26; G01N 2800/50; G01N 2800/52;
G01N 2800/54

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Universitätsmedizin der
Johannes Gutenberg-Universität Mainz
55131 Mainz (DE)**

(72) Inventors:
• **Ackermann, Maximilian
66679 Losheim am See (DE)**

• **Jonigk, Danny
30916 Isernhagen (DE)**
• **Schuppan, Detlef
55122 Mainz (DE)**
• **Surabattula, Rambabu
55128 Mainz (DE)**
• **Bockamp-Villamil, Ernesto
55126 Mainz (DE)**

(74) Representative: **Krauss, Jan
SKM-IP PartGmbB
Oberanger 45
80331 München (DE)**

(54) **BIOMARKERS FOR SYSTEMIC PULMONARY DISEASE (SPD) IN PARTICULAR SEVERE COVID19-DISEASE**

(57)    The present invention relates to a method for detecting and/or predicting the severity and/or chronicity of a systemic pulmonary disease (SPD) in a patient, comprising determining the expression of at least two markers selected from the group consisting of macrophage marker CD163, matricellular fibrosis marker thrombospondin-2 (Tsp2), and (cardiovascular) inflammation/repair marker growth differentiation factor 15 (GDF-15) in a sample obtained from said patient, optionally with complementation by insulin like growth factor binding protein 7 (IGFBP7). The method is particularly useful in case of SPDs such as influenza infection, such as, for example, influenza A virus infection, interstitial lung disease (ILD, IPF), acute respiratory distress syndrome (ARDS), and coronavirus infection, such as, for example, COVID19 and long COVID19.

EP 4 336 186 A1

## Description

The present invention relates to a method for detecting and/or predicting the severity and/or chronicity of a systemic pulmonary disease (SPD) in a patient, comprising determining the expression of at least two markers selected from the group consisting of macrophage marker CD163, matricellular fibrosis marker thrombospondin-2 (Tsp2), and (cardiovascular) inflammation/repair marker growth differentiation factor 15 (GDF-15) in a sample obtained from said patient, optionally with complementation by insulin like growth factor binding protein 7 (IGFBP7). The method is particularly useful in case of SPDs such as influenza infection, such as, for example, influenza A virus infection, interstitial lung disease (ILD, IPF), acute respiratory distress syndrome (ARDS), and coronavirus infection, such as, for example, COVID19 and long COVID19.

## Background of the invention

Systemic pulmonary disease (SPD) including severe COVID19-disease, other severe pulmonary infections with multisystem involvement (ARDS), and also active pulmonary fibrosis of various aetiologies are conditions that frequently lead to death or long-lasting sequelae, such as advanced fibrosis, so-called "long COVID19" or chronic fatigue syndrome (CFS). The associated multisystem disease involves mainly the lungs, liver, kidneys, the cardiovascular and the central nervous system.

Mandal S, et al. (in: ARC Study Group. 'Long-COVID': a cross-sectional study of persisting symptoms, biomarker and imaging abnormalities following hospitalisation for COVID-19. Thorax. 2021 Apr;76(4):396-398. doi: 10.1136/thoraxjnl-2020-215818. Epub 2020 Nov 10. PMID: 33172844; PMCID: PMC7661378) disclose that large numbers of people are being discharged from hospital following COVID-19 without assessment of recovery. In 384 patients (mean age 59.9 years; 62% male) followed a median 54 days post discharge, 53% reported persistent breathlessness, 34% cough and 69% fatigue. 14.6% had depression. In those discharged with elevated biomarkers, 30.1% and 9.5% had persistently elevated d-dimer and C reactive protein, respectively.

Avouac J, Cauvet A, Steelandt A, Shirai Y, Elhai M, Kuwana M, et al. (2020) Improving risk-stratification of rheumatoid arthritis patients for interstitial lung disease. PLoS ONE 15(5): e0232978. https://doi.org/10.1371/journal.pone.0232978 determined the performance of 3 circulating markers for the diagnosis and the progression of interstitial lung disease (ILD) associated with rheumatoid arthritis (RA). Serum concentrations of 3 circulating markers, lung epithelial-derived surfactant protein D (SPD), chemokine CCL-18 and "Krebs von den Lungen-6" glycoprotein (KL-6), were measured by ELISA in consecutive patients with established RA. The combination of KL-6 with SPD and CCL18 improved its diagnostic ability, with increased sensitivity from 68% to 77%, specificity from 83% to 97%. Increased KL-6 levels were independently associated with the presence of RA-ILD after the adjustment on other RA-ILD risk factors.

Ahmed DS, et al. (in:Coping With Stress: The Mitokine GDF-15 as a Biomarker of COVID-19 Severity. Front Immunol. 2022 Feb 16;13:820350. https://doi.org/10.3389/fimmu.2022.820350. PMID: 35251002; PMCID: PMC8888851) discuss the emergence of GDF-15 as a distinctive marker of viral infection severity, especially in the context of COVID-19.

WO 2022/017980 relates to a method for predicting the disease severity in a patient with COVID-19, said method comprising a) determining the level of GDF-15 in a sample from the patient with COVID-19, b) comparing the level determined in step a) to a reference, and predicting the disease severity in a patient with COVID-19. Also encompassed are methods for risk stratification, monitoring of disease progression and the risk prediction for hospital admission in a patient with COVID-19. Furthermore, methods are provided for predicting the need for intensive care, the risk for thrombosis, pulmonary embolism, hypoxia and mortality in a patient with COVID-19.

Myhre PL, et al. (in: Growth Differentiation Factor 15 Provides Prognostic Information Superior to Established Cardiovascular and Inflammatory Biomarkers in Unselected Patients Hospitalized With COVID-19. Circulation. 2020 Dec;142(22):2128-2137. doi: 10.1161/CIRCULATIONAHA.120.050360. Epub 2020 Oct 15. PMID: 33058695; PMCID: PMC7688084) disclose that growth differentiation factor-15 (GDF-15) is a cytokine upregulated in multiple pathological conditions where oxidative stress, endothelial dysfunction, tissue aging, and chronic inflammation are the hallmarks. GDF-15 has many sources of production, including cardiac and vascular myocytes, (myo-) fibroblasts, endothelial cells, adipocytes and macrophages in response to metabolic stress, to oncogenic transformation and the burden of proinflammatory cytokines or reactive oxygen species. Although the main sources of GDF-15 are extracardiac tissues, it has been shown to be elevated in many cardiac disorders. In experimental models of heart disease, GDF-15 release is induced after an ischemic insult and in pressure overload scenarios. Likewise, in recent years, an increasing body of evidence has emerged linking GDF-15 to the risk of mortality in acute coronary syndromes, atrial fibrillation and heart failure. Additionally, GDF-15 has been shown to add prognostic information beyond other conventional biomarkers such as natriuretic peptides and cardiac troponins. Further studies are needed to assess whether the incorporation of GDF-15 into clinical practice can improve cardiovascular outcomes.

Gomez-Rial J, et al. (in: Increased Serum Levels of sCD14 and sCD163 Indicate a Preponderant Role for Monocytes in COVID-19 Immunopathology. Front Immunol. 2020 Sep 23;11:560381. PMID: 33072099 PMCID: PMC7538662 DOI: 10.3389/fimmu.2020.560381) investigated two soluble markers of monocyte activation, sCD14 and sCD163, in COVID-19 patients, with the aim of characterizing their potential role in monocyte-macrophage disease immunopathology. sCD14 and sCD163 levels were significantly higher among COVID-19 patients, independently of ICU admission requirement, compared to the control group. They found a significant correlation between sCD14 levels and other inflammatory markers, particularly Interleukin-6, in the non-ICU patients group. sCD163 showed a moderate positive correlation with the time lapsed from admission to sampling, independently of severity. Treatment with corticoids showed an interference with sCD14 levels, whereas hydroxychloroquine and tocilizumab did not. The data suggest an important role for monocyte-macrophage activation in the development of immunopathology of COVID-19 patients.

Currently, there are no reliable non-invasive (blood or serum-based) biomarkers that allow to assess the overall activity, severity and prognosis of SPD including COVID-19, and especially the early response to therapeutic intervention in the acute disease, and also in the management of long-term consequences. It is an object of the present invention to provide highly predictive respective biomarkers and assays. Other objects will become apparent to the person of skill upon consideration of the following detailed description.

In a first aspect of the present invention, the above problem is solved by a method for detecting and/or predicting the severity and/or chronicity of a systemic pulmonary disease (SPD) in a patient, comprising

determining the expression of at least two markers selected from the group consisting of macrophage marker CD163, matricellular fibrosis marker thrombospondin-2 (Tsp2), and (cardiovascular) inflammation/repair marker growth differentiation factor 15 (GDF-15) in a sample obtained from said patient,
wherein an increase of the expression of the at least two markers detects and/or predicts the severity and/or chronicity of the systemic pulmonary disease (SPD) in the patient, when compared to a healthy control and/or a mild to moderate SPD control.

Preferred is the method according to the present invention, wherein the expression of all three markers is determined. Furthermore, the expression of the marker IGFBP7 may be determined.

Preferred is the method according to the present invention, wherein said SPD is selected from the group consisting of influenza infection, pulmonary fibrosis, idiopathic pulmonary fibrosis, interstitial lung disease (ILD), acute respiratory distress syndrome (ARDS), influenza A virus infection, and coronavirus infection, such as, for example, COVID19 and long COVID19.

The above problem is solved in a second aspect of the present invention by a method for diagnosing the severity and/or chronicity of a systemic pulmonary disease (SPD) in a patient, comprising performing the method according to the present invention, and further comprising the step of diagnosing the severity and/or chronicity of the systemic pulmonary disease (SPD) in the patient, wherein an increase of the expression of the at least two markers is diagnostic for a higher severity and/or longer chronicity of the systemic pulmonary disease (SPD) in the patient, when compared to a healthy control, an earlier sample form the same patient and/or a control sample. Preferred is the method according to the present invention, wherein the sample is obtained from a patient that is undergoing or has received treatment against an SPD.

Patients with long-term follow-up showed trajectories either with lower but continuously elevated biomarker levels, or with biomarker levels that normalized, in correlation to incomplete vs full recovery. The three selected biomarkers furthermore correlated with upregulated expression of the respective genes in lungs of patients with severe COVID19/SPD vs non-COVID/SPD lungs.

The above problem is solved in a third aspect of the present invention by a method for monitoring the severity and/or chronicity of a systemic pulmonary disease (SPD) in a patient, comprising performing the method according to the present invention as above, and further comprising the step of monitoring the severity and/or chronicity of the SPD in the patient based on a comparison with at least one determination as obtained when performing the method according to the present invention as above on an earlier sample form the same patient and/or on a control sample. Preferred is the method according to the present invention, wherein an increased level of expression is indicative of a more severe disease progression.

The above problem is solved in a fourth aspect of the present invention by a method for predicting the efficacy of a treatment of a systemic pulmonary disease (SPD) in a patient, comprising performing the method according to the present invention, and further comprising the step of predicting the efficacy of the treatment, wherein an decrease of the expression of the at least two markers is predictive for an efficient treatment of the systemic pulmonary disease (SPD) in the patient, when compared to a healthy control, an earlier sample form the same patient and/or a control sample.

In yet another aspect thereof, the present invention provides a method for identifying a compound for the prevention or treatment of a systemic pulmonary disease (SPD) in a subject, comprising the steps of administering at least one candidate compound to said subject, and determining the expression of at least two markers selected from the group consisting of macrophage marker CD163, matricellular fibrosis marker thrombospondin-2 (Tsp2), and (cardiovascular) inflammation/repair marker growth differentiation factor 15 (GDF-15) in a sample obtained from said subject in the presence of said at least one candidate compound and comparing it to the expression of said markers in a follow-up sample obtained from said subject or in the absence of said candidate compound and/or in a control sample, wherein a decrease of the expression of at least two of the markers identifies a compound for the prevention or treatment of a systemic pulmonary disease (SPD) in the subject. Preferred is the method according to the present invention, wherein the expression of all three markers is determined. Furthermore, the expression of the marker IGFBP7 may be determined separately or in addition to the other markers, yielding further information on disturbed immune metabolism.

In yet another aspect thereof, the present invention provides a method for testing the efficacy of a pharmaceutical composition or of pharmaceutical combinations, as well as for the efficacy of such pharmaceuticals in combination with other treatment strategies, for example, dietary, vitamin or trace element supplementation, physical therapy or exercise etc., for the treatment of SPD, and especially of the sequelae of SPD/COVID-19, e.g., long-COVID/CFS. Patients are tested for the present blood derived markers before, during and after treatment for these conditions. Effective therapies are then characterized by (significant) decreases of these markers individually or in combination. Preferably, using the present method, clinical therapeutic effects that are not apparent at the start of therapy can be predicted well in advance of detectable clinical benefit.

In another aspect thereof, the biomarker combinations will permit an early assessment of therapeutic efficacy of such measures, especially their combinations, allowing a rapid assessment of efficacy of such intervention, and in a personalized way, which is impossible using current methodologies based on single serological or physiological parameters that need to be applied in conventional clinical studies.

In yet another aspect thereof, the present invention provides a method for producing a pharmaceutical composition comprising the step of admixing at least one compound for the prevention or treatment of a systemic pulmonary disease (SPD) as identified according to the present invention as above with at least one suitable pharmaceutically acceptable carrier or diluent.

The present invention provides a pharmaceutical composition comprising at least one compound for the prevention or treatment of a systemic pulmonary disease (SPD) as identified according to the present invention as above, together with at least one pharmaceutically acceptable carrier or diluent.

The above problem is solved in a ninth aspect of the present invention by the at least one compound for the prevention or treatment of a systemic pulmonary disease (SPD) as identified according to the present invention or the pharmaceutical composition according to the present invention for use in medicine, preferably for use in the prevention or treatment of a systemic pulmonary disease (SPD), such as influenza infection, such as, for example, influenza A virus infection, interstitial lung disease (ILD), acute respiratory distress syndrome (ARDS), and coronavirus infection, such as, for example, COVID19 and long COVID19 in a patient.

In yet another aspect thereof, the present invention provides a method for preventing or treating a systemic pulmonary disease (SPD), such as influenza infection, such as, for example, influenza A virus infection, interstitial lung disease (ILD), acute respiratory distress syndrome (ARDS), and coronavirus infection, such as, for example, COVID19 and long COVID19 in a patient, comprising administering an effective amount of the at least one compound as identified according to the present invention or the pharmaceutical composition according to the present invention to said patient. Preferred is the use or the method according to the present invention, wherein patient is undergoing or has received additional treatment against the SPD.

In yet another aspect thereof, the present invention provides a kit, such as, for example, a diagnostic kit or a kit in combination with a therapeutic principle, as described above, (companion biomarker to guide a personalized medicine) comprising materials for performing the method for detecting and/or predicting the severity and/or chronicity of a systemic pulmonary disease (SPD) in a patient according to the present invention, the method for diagnosing the severity and/or chronicity of a systemic pulmonary disease (SPD) in a patient according to the present invention, the method for monitoring the severity and/or chronicity of a systemic pulmonary disease (SPD) in a patient according to the present invention, the method for predicting the efficacy of a treatment of a systemic pulmonary disease (SPD) in a patient according to the present invention, the method for identifying a compound for the prevention or treatment of a systemic pulmonary disease (SPD) in a subject according to the present invention, or the method for preventing or treating a systemic pulmonary disease (SPD) according to the present invention.

In yet another aspect thereof, the present invention provides the use of the kit according to the present invention in performing the method for detecting and/or predicting the severity and/or chronicity of a systemic pulmonary disease (SPD) in a patient according to the present invention, the method for diagnosing the severity and/or chronicity of a systemic pulmonary disease (SPD) in a patient according to the present invention, the method for monitoring the severity and/or chronicity of a systemic pulmonary disease (SPD) in a patient according to according to the present invention, the method for predicting the efficacy of a treatment of a systemic pulmonary disease (SPD) in a patient according to the present invention, the method for identifying a compound for the prevention or treatment of a systemic pulmonary disease (SPD) in a subject according to the present invention, or the method for preventing or treating a systemic pulmonary disease (SPD) according to the present invention.

As mentioned above, the present invention in a first aspect relates to a method for detecting and/or predicting the severity and/or chronicity of a systemic pulmonary disease (SPD) in a patient, comprising determining the expression of at least two markers selected from the group consisting of macrophage marker CD163, matricellular fibrosis marker thrombospondin-2 (Tsp2), and (cardiovascular) inflammation/repair marker growth differentiation factor 15 (GDF-15) in a sample obtained from said patient, wherein an increase of the expression of the at least two markers detects and/or predicts the severity and/or chronicity of the systemic pulmonary disease (SPD) in the patient, when compared to a healthy control and/or a mild to moderate SPD control.

In the context of the present invention, the inventors used and optimized commercially available ELISA tests for the markers CD 163 and GDF15 (R&D systems). An ELISA for Tsp-2 based on the inventors' own recombinant expression of the protein and two commercial antibodies (mouse monoclonal and goat polyclonal) was established and validated.

Serum/plasma samples from 87 hospitalized patients with well-defined diagnosis of COVID19 and other etiologies of SPD in the acute phase of their diseases and in early follow-up up to 4 weeks were collected. Sub-cohorts of patients who survived with complete remission or long-term sequelae, especially long-COVID19 syndrome, were followed for up to 4 months. In those patients who did not survive due to their COVID19 disease or SPD the overall gene expression in lungs of was determined from post-mortem lungs by transcriptome analysis and correlated to the serum/plasma levels of our markers CD163, Tsp2 and GDF15.

The present invention is generally based on the use of the biomarkers CD163, Tsp2, and GDF-15, the expression and especially combination of which can surprisingly be used in order to detect and/or predict the severity and/or chronicity of a systemic pulmonary disease (SPD) in a patient, when compared to a healthy controls and/or mild to moderate SPD controls.

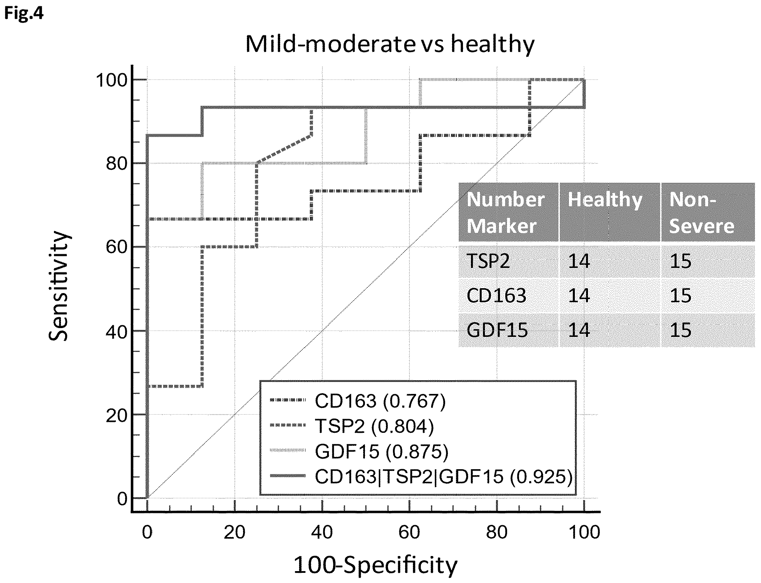

The measurements as obtained, such as the increases are used to calculate biomarker algorithms for an optimal prediction, as exemplified in the examples below and shown in the figures, in particular figures 4 to 6. With the algorithms, a slightly different weight is given to the different markers, and the algorithms may change slightly depending on the number of patients as studied, and the SPD etiologies - for example a 5%, 10%, or 20% change of the numbers before serum levels (in ng/ml) depending on different patient age groups, BMIs or ethnicities. In view of the present disclosure, establishing the algorithm and adjusting it to the circumstances of the assay and the markers as chosen is well within the regular skill of the skilled person.

As an example, serum/plasma markers CD163, Tsp2 and GDF15 predicted the course of mild-moderate COVID19 vs healthy controls with areas under receiver operating curves (AUROCs) of 0.767-0.875, and severe COVID19 vs mild to moderate COVID19 with AUROCs of 0.692-0.831. Similar results were obtained for other SPDs, like influenza. The combination of the three biomarkers is a superior predictor of disease as compared to the single biomarker levels, reaching AUROCs of 0.925 and 0.869, respectively.

The present invention uses at least two of the markers in combination, preferably three, i.e., in a combination selected from CD163 and Tsp2, CD163 and GDF15, Tsp2 and GDF15, and CD163 and Tsp2 and GDF15. Preferred is a method according to the present invention, wherein furthermore the expression of the marker IGFBP7 is determined.

In the context of the present invention, the term "CD163" generally refers to the CD163 molecule protein or Scavenger Receptor Cysteine-Rich Type 1 Protein M130. The term shall encompass the human version of this protein, but also its homologs, in particular in mouse or rat. Information on CD163 can be derived from the UniProt webpage under the accession number Q86VB7. All isoforms of the protein and its major functional protein domains shall also be encompassed by the present invention.

In the context of the present invention, the term "Tsp2" generally refers to the Thrombospondin 2 molecule protein. The term shall encompass the human version of this protein, but also its homologs, in particular in mouse or rat. Information on Tsp2 can be derived from the UniProt webpage under the accession number P35442. All isoforms of the protein and its major functional protein domains shall also be encompassed by the present invention.

In the context of the present invention, the term "GDF15" generally refers to the Growth/differentiation factor 15. The term shall encompass the human version of this protein, but also its homologs, in particular in mouse or rat. Information on GDF15 can be derived from the UniProt webpage under the accession number Q99988. All isoforms of the protein and its major functional protein domains shall also be encompassed by the present invention.

In the context of the present invention, the term "IGFBP7" generally refers to the insulin-like growth factor-binding protein 7 molecule protein. The term shall encompass the human version of this protein, but also its homologs, in particular in mouse or rat. Information on IGFBP7 can be derived from the UniProt webpage under the accession number Q16270. All isoforms of the protein and its major functional protein domains shall also be encompassed by the present invention.

In context of the invention, the terms CD163, GDF15, Tsp-2, and IGFBP7 shall also include proteins, variants or fragments that exhibit an amino acid with a sequence identity of at least 80, 85, 86, 87, 88, 89, 90, 91, 92, 93, 94, 95, 96, 97, 98, 99 or 100% compared to the amino acid sequences of human CD163, GDF15, Tsp-2, or IGFBP7, respectively, as disclosed herein. Fragments of CD163, GDF15, Tsp-2, or IGFBP7 or their variants, shall preferably have a length of at least 30, 40, 50, 80, 100, 150, 200, 300 or more amino acids.

As used herein, the terms "identical" or percent "identity", when used anywhere herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using sequence comparison algorithms, or by manual alignment and visual inspection (see, e.g., NCBI web site). In particular for amino acid identity, those using BLASTP 2.2.28+ with the following parameters: Matrix: BLOSUM62; Gap Penalties: Existence: 11, Extension: 1; Neighboring words threshold: 11; Window for multiple hits: 40.

In the context of the present invention, the term "determining the expression of a marker" generally refers to any suitable method to detect or determine the presence and/or changes in the presence of a marker as detected according to the present invention. Detecting or determining the expression can be done directly or indirectly, i.e. either the transcription product of the respective gene(s) is determined (e.g. mRNA abundance) or the product of the translation itself is directly determined (e.g. protein abundance) in a sample.

Determining the expression of a marker preferably comprises a marker-specific antibody, an immunoassay, such as, for example, an ELISA, or the analysis of the transcription of the marker, such as, for example, using chip analysis. Also fragments of the markers may be detected, for example when using an analysis comprising mass spectrometry. The markers may be suitably labelled, e.g., by fluorescence or other markers.

In the method of the present invention, any suitable sample comprising the makers as detected may be used. The sample may be pre-conditioned, e.g., serum is obtained from whole blood. Preferred is a method according to the present invention, wherein the sample obtained from said patient and/or said control sample is a blood, serum or plasma sample or mixtures thereof.

Particularly preferred assays are determining plasma GDF15, CD163, and TSP2 by enzyme linked immunosorbent assay (ELISA) or determining the amount of mRNA.

In the context of the invention, the expression of the biomarkers CD163, Tsp2, and GDF-15, is used in order to detect and/or predict the severity and/or chronicity of a systemic pulmonary disease (SPD) in a patient. SPD covers a variety of pulmonary diseases, mostly including fibrosis, sclerosis and scarring. Examples for SPDs are selected from influenza infection, such as, for example, influenza A virus infection, interstitial lung disease (ILD) including (idiopathic) pulmonary fibrosis, acute respiratory distress syndrome (ARDS), and coronavirus infection, such as, for example, COVID19 and long COVID19. Interstitial lung disease (ILD), for example, is an umbrella term used for a large group of diseases that also cause scarring (fibrosis) of the lungs. The scarring causes stiffness in the lungs which makes it difficult to breathe and get oxygen to the bloodstream. Lung damage from ILDs is often irreversible and gets worse over time. The present invention is also particularly effective in detecting and/or predicting the severity and/or chronicity of long COVID.

Preferred is the method according to the present invention, wherein the detection and/or prediction of the severity and/or chronicity comprises at least one of i) distinguishing severe COVID19 disease or ARDS from milder SPD, and/or from moderate to mild COVID19 disease, and ii) predicting severe COVID19 disease or ARDS from milder SPD, and/or from moderate to mild COVID19 disease in the patient. See examples and Figures, below.

Moreover, application of the method is equally suitable for detection, severity prediction, prediction of the efficacy of (novel) therapeutics or therapeutic measures, and follow-up of other SPD including fibrotic lung diseases, especially with the aim of a personalized patient management and medicine.

Preferred is the method according to the present invention, wherein the detecting and/or predicting the severity and/or chronicity comprises at least one of i) differentiation of mild-moderate COVID19 disease from healthy controls within the first two days of admission of the patient, and ii) differentiating an emerging severe from a mild-moderate COVID19 disease within the first two days of admission of the patient.

Preferred is the method according to the present invention, wherein the detection and/or prediction of the severity and/or chronicity comprises differentiating a severe vs mild evolution of long-COVID syndrome or other SPD in said patient based on an increase of the expression of the markers CD163 and TSP2, or CD163, Tsp2 and GDF15, or CD163, Tsp2, GDF15 and IGFBP7, such as, for example, a re-spiking of the markers in the patient.

The methods and assays that are determining and detecting the markers GDF15, CD163, and TSP2 may also be used in a diagnostic context. Thus, the present invention provides a method for diagnosing the severity and/or chronicity of a systemic pulmonary disease (SPD) in a patient, comprising performing the method according to present invention as above, and further comprising the step of diagnosing the severity and/or chronicity of the systemic pulmonary disease (SPD) in the patient, wherein an increase of the expression of the combination of at least two markers creates an algorithm that is diagnostic for a higher severity and/or longer chronicity of the systemic pulmonary disease (SPD) in the patient, when compared to a healthy control, an earlier sample from the same patient and/or a control sample. Of particular interest is the method according to the present invention, wherein the sample is obtained from a patient who is undergoing or has received treatment against an SPD. The markers are used to identify, whether the treatment has an effect on the respective SPD (companion biomarkers). A preferred embodiment of this method also comprises monitoring (see below).

Serum/plasma markers CD163, Tsp2 and GDF15 predicted the course of mild-moderate COVID19 vs healthy controls with areas under receiver operating curves (AUROCs) of 0.767- 0.875, and severe COVID19 vs mild to moderate COVID19 with AUROCs of 0.692-0.831. Similar results were obtained for SPD. The combination of two of these biomarkers is a superior predictor of disease compared to the single biomarker levels., reaching even higher AUROC values.

The combination of the three biomarkers is an even superior predictor of disease as compared to the dual biomarker assay, reaching preferably AUROCs of 0.925 and 0.869, respectively. The marker IGFBP adds additional information on disease severity and prognosis.

Patients with long-term follow-up showed trajectories either with lower but continuously elevated biomarker levels, or with biomarker levels that normalized, in correlation to incomplete vs full recovery. The three selected biomarkers CD163, Tsp2 and GDF15 furthermore correlated with upregulated expression of the respective genes in lungs of patients with severe SPD/COVID19 vs non-SPD/COVID lungs.

The present invention further provides a method for monitoring the severity and/or chronicity of a systemic pulmonary disease (SPD) in a patient, comprising performing the method according to the present invention, and further comprising the step of monitoring the severity and/or chronicity of the systemic pulmonary disease (SPD) in the patient based on a comparison with at least one determination as obtained when performing the method according to the present invention on an earlier sample form the same patient and/or on a control sample.

This method thus comprises the methods for identifying or diagnosing as above on a sample derived from a patient during disease evolution and especially the course of a treatment, and comparing the results with a control sample, in particular a sample from the patient taken at the onset of disease or admission, or at the time before the treatment of the SPD.

Preferred is a method according to the present invention, wherein an increased level of expression is indicative of a more severe disease progression.

Further preferred is a method for predicting the efficacy of a treatment of a systemic pulmonary disease (SPD) in a patient, comprising performing the method for monitoring according to the invention as above, and further comprising the step of predicting the efficacy of the treatment, wherein an decrease of the expression of the at least two markers is predictive for an efficient treatment of the systemic pulmonary disease (SPD) in the patient, when compared to a healthy control, an earlier sample form the same patient and/or a control sample. Moreover, the decrease of the biomarker levels is correlated with the reconvalescence from the disease and correlates with efficacy of a therapeutic intervention as early as the intervention affects the underlying pathophysiology, since the biomarkers reflect the dynamics of the pathological processes in contrast to e.g. conventional imaging via CT or MRT that are crude cross-sectional analysis methods, whereas the biomarkers as used herein react to an effective therapy within a few days or weeks.

Yet another important aspect of the present invention relates to a method for identifying a compound for the prevention or treatment of a systemic pulmonary disease (SPD) in a subject, comprising the steps of administering at least one candidate compound to said subject, and determining the expression of at least two markers selected from the group consisting of macrophage marker CD163, matricellular fibrosis marker thrombospondin-2 (Tsp2), and (cardiovascular) inflammation/repair marker growth differentiation factor 15 (GDF-15) in a sample obtained from said subject in the presence of said at least one candidate compound and comparing it to the expression of said markers in a sample obtained from said subject in the absence of said at least one candidate compound and/or in a control sample, wherein an decrease of the expression of the at least two markers identifies a compound for the prevention or treatment of a systemic pulmonary disease (SPD) in the subject.

In this aspect, the method of the present invention is used to identify or screen for pharmaceutical compounds that are effective in the treatment of the SPD, as reflected by a change in the expression of the markers as detected and/or determined in the context of the present invention.

As above, in the method of the present invention, any suitable sample comprising the markers as detected may be used. Sample may be pre-conditioned, e.g. serum is obtained from whole blood. Preferred is a method according to the present invention, wherein the sample obtained from said patient and/or said control sample is a blood, serum or plasma sample or mixtures thereof. Preferably, the expression of all three markers is determined, and optionally also the expression of the marker IGFBP7 is determined.

Preferably, determining or detecting the expression of a marker comprises a marker-specific antibody, an immunoassay, such as, for example, an ELISA, but also other technologies to detect the target proteins, such as aptamer or mass spectrometry-based technologies, or the analysis of the transcription of the marker, such as, for example, using chip analysis.

Suitable candidate compounds can be selected from pharmaceutically approved compounds (e.g, for a re-purpose), derived from compound libraries, natural compounds, small molecules (usually smaller than 500 Da), peptides, proteins, and the like.

Yet another important aspect of the present invention relates to a method for producing a pharmaceutical composition comprising the step of admixing at least one compound for the prevention or treatment of a systemic pulmonary disease (SPD) as identified according to the present invention with at least one suitable pharmaceutically acceptable carrier or diluent, and/or excipient.

Yet another aspect of the present invention then relates to a pharmaceutical composition, comprising at least one compound for the prevention or treatment of a systemic pulmonary disease (SPD) as identified according to the present invention, together with a pharmaceutically acceptable carrier and/or auxiliary agent.

As used herein the language "pharmaceutically acceptable" carrier, stabilizer or excipient is intended to include any and all solvents, solubilizers, fillers, stabilizers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of the invention is formulated to be compatible with its in-tended route of administration. Examples of routes of administration include parenteral, e.g., intrathecal, intra-arterial, intravenous, intradermal, subcutaneous, oral, transdermal (topical) and transmucosal administration.

Pharmaceutical compositions as used may comprise a pharmaceutically acceptable carrier. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal $Si0_2$), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti-oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for respective compounds, for example topical, and will readily be able to choose suitable pharmaceutically acceptable carriers or excipients, depending, e.g., on the formulation and administration route of the pharmaceutical composition.

Yet another aspect of the present invention then relates to the at least one compound for the prevention or treatment of a systemic pulmonary disease (SPD) as identified according to the present invention or the pharmaceutical composition according to the present invention for use in medicine, preferably for use in the prevention or treatment of a systemic pulmonary disease (SPD), such as influenza infection, such as, for example, influenza A virus infection, interstitial lung disease (ILD), acute respiratory distress syndrome (ARDS), and coronavirus infection, such as, for example, COVID19 and long COVID19 in a patient.

Yet another aspect of the present invention then relates to a method for preventing or treating a systemic pulmonary disease (SPD), such as influenza infection, such as, for example, influenza A virus infection, interstitial lung disease (ILD), acute respiratory distress syndrome (ARDS), and coronavirus infection, such as, for example, COVID19 and long COVID19 in a patient, comprising administering an effective amount of the at least one compound as identified according to the present invention or the pharmaceutical composition according to the present invention to said patient.

By "treatment" or "treating" is meant any treatment of a disease or disorder, in a mammal, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. Methods for preventing or treating SPD are known to the person of skill, and are disclosed in the art. An effective amount of a compound or composition causes at least one of these effects.

Preferred is the method according to the present invention, wherein the patient is human or can also be preceded by an experimental animal. Further preferred is the method according to the present invention, wherein patient is undergoing or has received additional treatment against the SPD.

Yet another aspect of the present invention then relates to a kit, such as, for example, a diagnostic or therapeutic kit, comprising materials for performing the method for detecting and/or predicting the severity and/or chronicity of a systemic pulmonary disease (SPD) in a patient according to the present invention, the method for diagnosing the severity and/or chronicity of a systemic pulmonary disease (SPD) in a patient according to the present invention, the method for monitoring the severity and/or chronicity of a systemic pulmonary disease (SPD) in a patient according to the present invention, the method for predicting the efficacy of a treatment of a systemic pulmonary disease (SPD) in a patient according to the present invention, the method for identifying a compound for the prevention or treatment of a systemic pulmonary disease (SPD) in a subject according to the present invention, or the method for preventing or treating a systemic pulmonary disease (SPD) according to the present invention.

In the context of the present invention, materials for performing the methods as described may be selected from buffers and manuals for the respective assay, such as a set of antibodies that is specific for determining at least one of plasma GDF15, CD163, TSP2 (and IGFBP7) by enzyme linked immunosorbent assay (ELISA).

Yet another aspect of the present invention then relates to the use of the kit according to the present invention in performing the method for detecting and/or predicting the severity and/or chronicity of a systemic pulmonary disease (SPD) in a patient according to the present invention, the method for diagnosing the severity and/or chronicity of a systemic pulmonary disease (SPD) in a patient according to the present invention, the method for monitoring the severity and/or chronicity of a systemic pulmonary disease (SPD) in a patient according to the present invention, the method for predicting the efficacy of a treatment of a systemic pulmonary disease (SPD) in a patient according to the present invention, the method for identifying a compound for the prevention or treatment of a systemic pulmonary disease (SPD) in a subject according to the present invention, or the method for preventing or treating a systemic pulmonary disease (SPD) according to the present invention.

The present invention preferably relates to the following items:

Item 1. A method for detecting and/or predicting the severity and/or chronicity of a systemic pulmonary disease (SPD) in a patient, comprising

> determining the expression of at least two markers selected from the group consisting of macrophage marker CD163, matricellular fibrosis marker thrombospondin-2 (Tsp2), and (cardiovascular) inflammation/repair marker growth differentiation factor 15 (GDF-15) in a sample obtained from said patient,
> wherein an increase of the expression of the at least two markers detects and/or predicts the severity and/or chronicity of the systemic pulmonary disease (SPD) in the patient, when compared to a healthy control and/or a mild to moderate SPD control.

Item 2. The method according to Item 1, wherein the sample obtained from said patient and/or said control sample is a blood, serum or plasma sample or mixture thereof.

Item 3. The method according to Item 1 or 2, wherein the expression of all three markers is determined.

Item 4. The method according to any one of Items 1 to 3, wherein furthermore the expression of the marker insulin like growth factor binding protein 7 (IGFBP7) is determined.

Item 5. The method according to any one of Items 1 to 4, wherein determining the expression of a marker comprises a marker-specific antibody, an immunoassay, such as, for example, an ELISA, or protein determination with specific DNA aptamers. Or mass spectrometry technology, or the analysis of the transcription of the marker, such as, for example, using chip analysis.

Item 6. The method according to any one of Items 1 to 5, wherein said SPD is selected from the group consisting of influenza infection, pulmonary fibrosis, idiopathic pulmonary fibrosis, interstitial lung disease (ILD), acute respiratory distress syndrome (ARDS), influenza A virus infection, and coronavirus infection, such as, for example, COVID19 and long COVID19.

Item 7. The method according to any one of Items 1 to 6, wherein the detection and/or prediction of the severity and/or chronicity comprises at least one of i) distinguishing severe COVID19 disease or ARDS from milder SPD, and/or from moderate to mild COVID19 disease, and ii) predicting severe COVID19 disease or ARDS from milder SPD, and/or from moderate to mild COVID19 disease in the patient.

Item 8. The method according to any one of Items 1 to 6, wherein the detecting and/or predicting the severity and/or chronicity comprises at least one of i) differentiation of mild-moderate COVID19 disease from healthy controls within the first two days of admission of the patient, and ii) differentiating an emerging severe from a mild-moderate COVID19 disease within the first two days of admission of the patient.

Item 9. The method according to any one of Items 1, 2, 5 or 6, wherein the detection and/or prediction of the severity and/or chronicity comprises differentiating a severe vs mild evolution of long-COVID syndrome in said patient based on an increase of the expression of the markers CD163 and TSP2, such as, for example, a re-spiking of the markers in the patient.

Item 10. A method for diagnosing the severity and/or chronicity of a systemic pulmonary disease (SPD) in a patient, comprising performing the method according to any one of Items 1 to 9, and further comprising the step of diagnosing the severity and/or chronicity of the systemic pulmonary disease (SPD) in the patient, wherein an increase of the expression of the at least two markers is diagnostic for a higher severity and/or longer chronicity of the systemic pulmonary disease (SPD) in the patient, when compared to a healthy control, an earlier sample form the same patient and/or a control sample.

Item 11. The method according to any one of Items 1 to 10, wherein the sample is obtained from a patient that is undergoing or has received treatment against an SPD.

Item 12. A method for monitoring the severity and/or chronicity of a systemic pulmonary disease (SPD) in a patient, comprising performing the method according to Item 10 or 11, and further comprising the step of monitoring the severity and/or chronicity of the systemic pulmonary disease (SPD) in the patient based on a comparison with at least one determination as obtained when performing the method according to Item 10 or 11 on an earlier sample form the same patient and/or on a control sample.

Item 13. The method according to any one of Items 10 to 12, wherein an increased level of expression is indicative of a more severe disease progression.

Item 14. A method for predicting the efficacy of a treatment of a systemic pulmonary disease (SPD) in a patient, comprising performing the method according to Item 11, and further comprising the step of predicting the efficacy of the treatment, wherein an decrease of the expression of the at least two markers is predictive for an efficient treatment of the systemic pulmonary disease (SPD) in the patient, when compared to a healthy control, an earlier sample form the same patient and/or a control sample.

Item 15. A method for identifying a compound for the prevention or treatment of a systemic pulmonary disease (SPD) in a subject, comprising the steps of administering at least one candidate compound to said subject, and determining the expression of at least two markers selected from the group consisting of macrophage marker CD163, matricellular fibrosis marker thrombospondin-2 (Tsp2), and (cardiovascular) inflammation/repair marker growth differentiation factor 15 (GDF-15) in a sample obtained from said subject in the presence of said at least one candidate compound and comparing it to the expression of said markers in a sample obtained from said subject in the absence of said at least one candidate compound and/or in a control sample, wherein an decrease of the expression of the at least two markers identifies a compound for the prevention or treatment of a systemic pulmonary disease (SPD) in the subject.

Item 16. The method according to Item 15, wherein the sample obtained from said patient and/or said control sample is a blood, serum or plasma sample or mixture thereof.

Item 17. The method according to Item 15 or 16, wherein the expression of all three markers is determined.

Item 18. The method according to any one of Items 15 to 17, wherein furthermore the expression of the marker IGFBP7 is determined.

Item 19. The method according to any one of Items 15 to 18, wherein determining the expression of a marker comprises a marker-specific antibody, an immunoassay, such as, for example, an ELISA, or the analysis of the transcription of the marker, such as, for example, using chip analysis.

Item 20a. Method for producing a pharmaceutical composition comprising the step of admixing at least one compound for the prevention or treatment of a systemic pulmonary disease (SPD) as identified according to any one of Items 15 to 19 with at least one suitable pharmaceutically acceptable carrier or diluent.

Item 20. A pharmaceutical composition comprising at least one compound for the prevention or treatment of a systemic pulmonary disease (SPD) as identified according to any one of Items 15 to 19, together with at least one pharmaceutically acceptable carrier or diluent.

Item 21. The at least one compound for the prevention or treatment of a systemic pulmonary disease (SPD) as identified according to any one of Items 15 to 19 or the pharmaceutical composition according to Item 20 for use in medicine, preferably for use in the prevention or treatment of a systemic pulmonary disease (SPD), such as influenza infection, such as, for example, influenza A virus infection, interstitial lung disease (ILD), acute respiratory distress syndrome (ARDS), and coronavirus infection, such as, for example, COVID19 and long COVID19 in a patient.

Item 22. A method for preventing or treating a systemic pulmonary disease (SPD), such as influenza infection, such as, for example, influenza A virus infection, interstitial lung disease (ILD), acute respiratory distress syndrome (ARDS), and coronavirus infection, such as, for example, COVID19 and long COVID19 in a patient, comprising administering an effective amount of the at least one compound as identified according to any one of Items 15 to 19 or the pharmaceutical composition according to claim 20 to said patient.

Item 23. The method according to Item 21 or 22, wherein the patient is selected from a mouse or human.

Item 24. The method according to any one of Items 21 to 23, wherein patient is undergoing or has received additional treatment against the SPD.

Item 25. A kit, such as, for example, a diagnostic or therapeutic kit, comprising materials for performing the method for detecting and/or predicting the severity and/or chronicity of a systemic pulmonary disease (SPD) in a patient according to any one of Items 1 to 9 and 11, the method for diagnosing the severity and/or chronicity of a systemic pulmonary disease (SPD) in a patient according to any one of Items 10 or 11, the method for monitoring the severity and/or chronicity of a systemic pulmonary disease (SPD) in a patient according to Item 12 or 13, the method for predicting the efficacy of a treatment of a systemic pulmonary disease (SPD) in a patient according to Item 14, the method for identifying a compound for the prevention or treatment of a systemic pulmonary disease (SPD) in a subject according to any one of Items 15 to 19, or the method for preventing or treating a systemic pulmonary disease (SPD) according to any one of claims 22 to 24.

Item 26. Use of the kit according to Item 25 in performing the method for detecting and/or predicting the severity and/or chronicity of a systemic pulmonary disease (SPD) in a patient according to any one of Items 1 to 9 and 11, the method for diagnosing the severity and/or chronicity of a systemic pulmonary disease (SPD) in a patient according to any one of Item 10 or 11, the method for monitoring the severity and/or chronicity of a systemic pulmonary disease (SPD) in a patient according to claim 12 or 13, the method for predicting the efficacy of a treatment of a systemic pulmonary disease (SPD) in a patient according to Item 14, the method for identifying a compound for the prevention or treatment of a systemic pulmonary disease (SPD) in a subject according to any one of Items 15 to 19, or the method for preventing or treating a systemic pulmonary disease (SPD) according to any one of Items 22 to 24.

The present invention will now be further described in the following examples with reference to the accompanying figures and sequences, nevertheless, without being limited thereto. For the purposes of the present invention, all references as cited herein are incorporated by reference in their entireties.

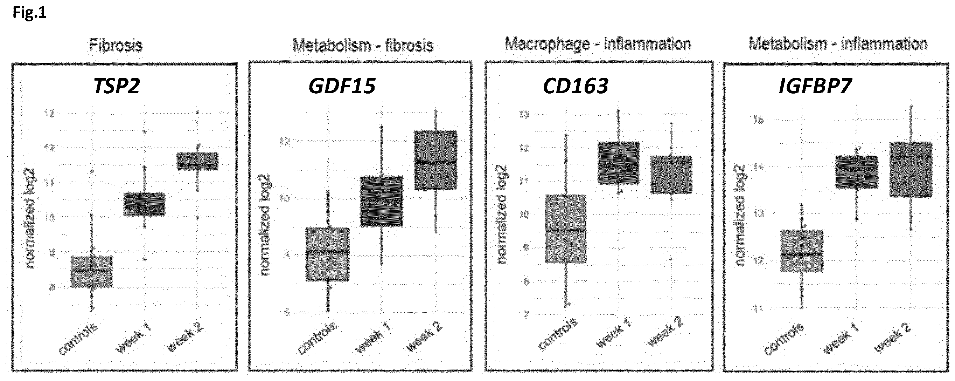

Figure 1: Pulmonary expression of key disease activity markers from post-mortem COVID-19 lungs. Identification and quantification of prominent COVID19-disease related genes for TSP2, GDF5, CD163 and IGFBP7 that are highly upregulated in severe COVID19 infection by nanostring-mass spectrometry.

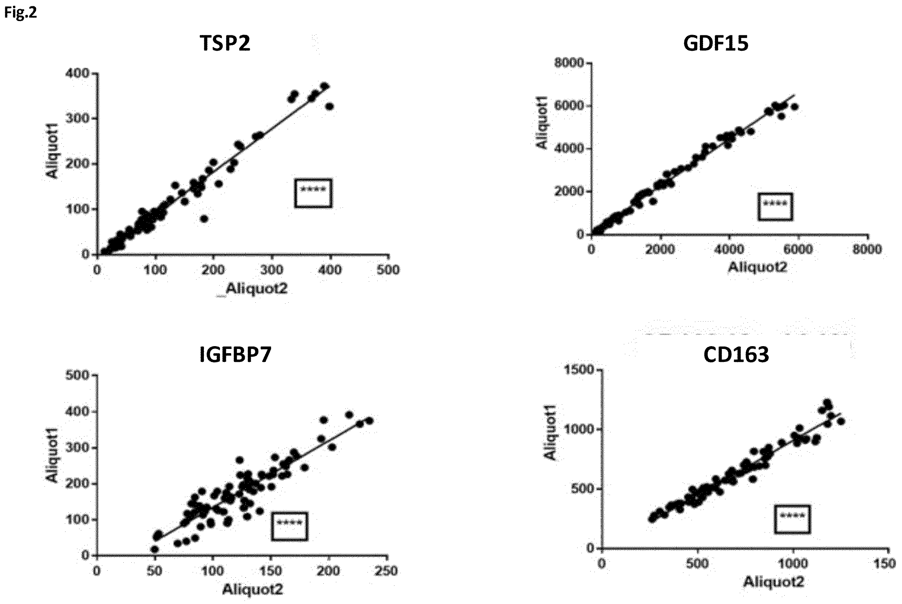

Figure 2: Development of in-house ELISAs to measure serum/plasma levels key disease related protein markers. ELISA assays for serum/plasma levels of CD163, IGFBP7 (insulin-like growth factor binding protein 7), Thrombospondin-2 (TSP2) and growth differentiation factor 15 (GDF15) were developed in-house based on sandwich designs using a monoclonal capture antibody and a horseradish peroxidase-labelled detection antibody. The figure shows the comparison of two independent plasma aliquots that were measured on different days in patients with mild to severe pulmonary multisystem diseases. Overall intra- and interassay variations in sets of normal and pathological samples were maximally 10% and 13% with 3 and 5 comparisons, respectively.

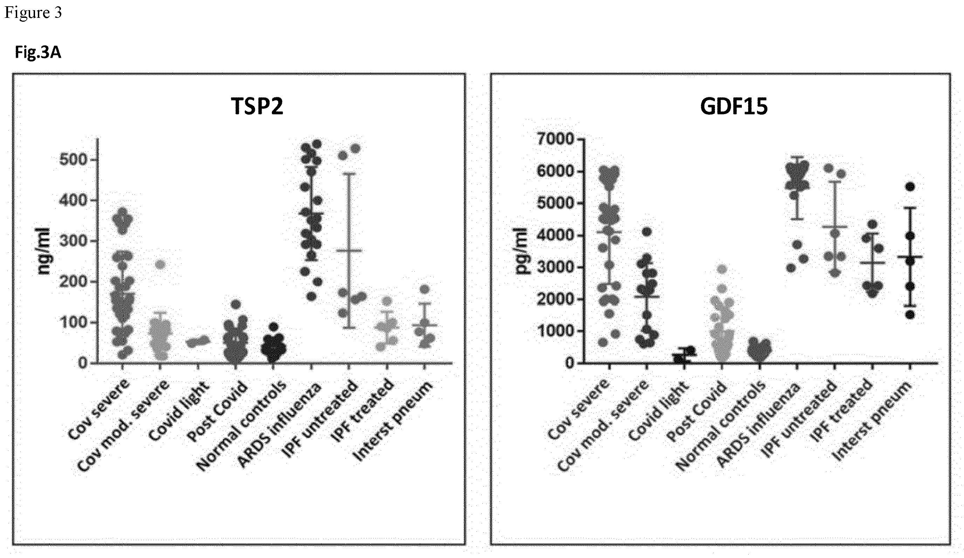

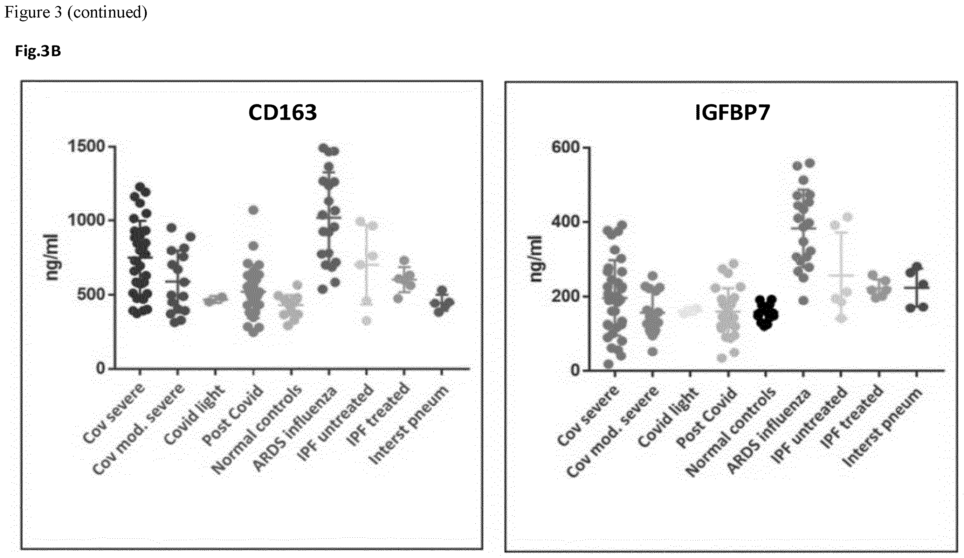

Figure 3: Differentiation of pulmonary-systemic disease severity by select serum/plasma markers. The three disease-domain specific serum/plasma markers TSP2, GDF15 and CD163 according to the invention show an excellent predictive value to separate severe COVID19 disease and acute respiratory distress syndrome (ARDS) from milder pulmonary-systemic diseases, and from moderate to mild COVID19 disease.

Figure 4: A three-serum/plasma-biomarker based calculation results in an algorithm that improves early diagnosis of mild-moderate COVID19 disease from healthy controls. The three disease-domain specific serum/plasma markers TSP2, GDF15 and CD163 according to the invention allow differentiation of mild-moderate COVID19 disease within the first two days of admission.

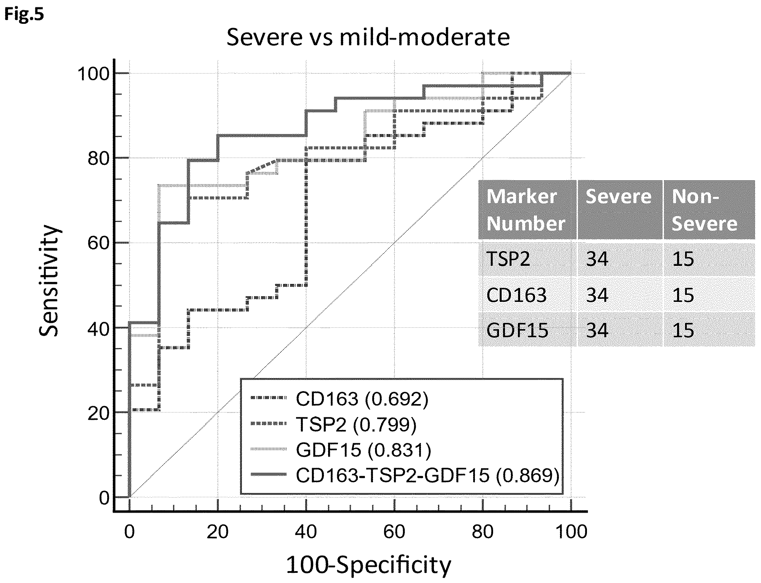

Figure 5: A three-serum/plasma-biomarker algorithm allows early prediction of a severe vs a mild-moderate COVID19 disease course. The three disease-domain specific serum/plasma markers TSP2, GDF15 and CD163 allow to differentiate emerging severe from mild-moderate COVID19 disease within the first two days of admission.

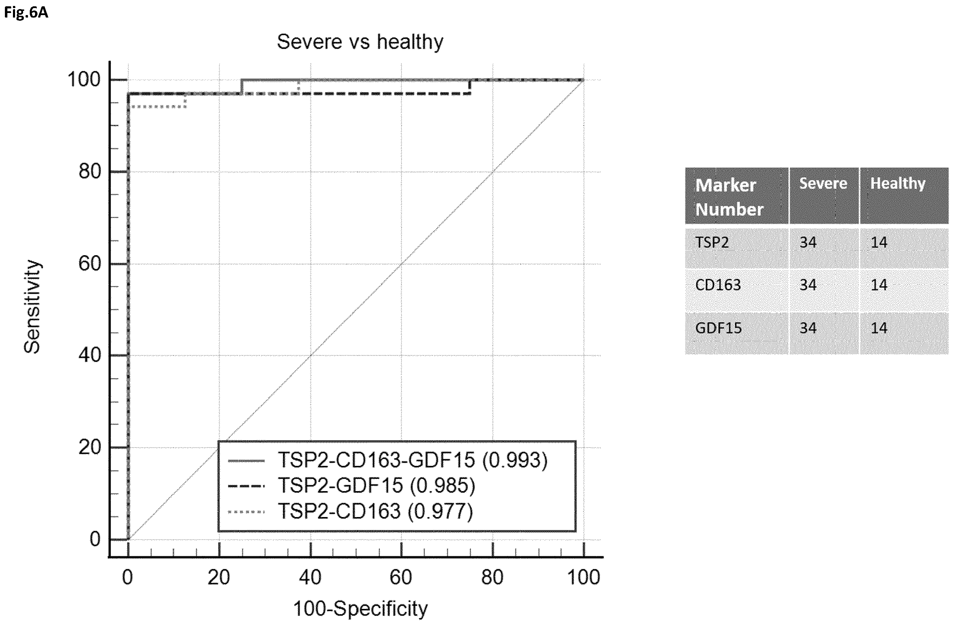

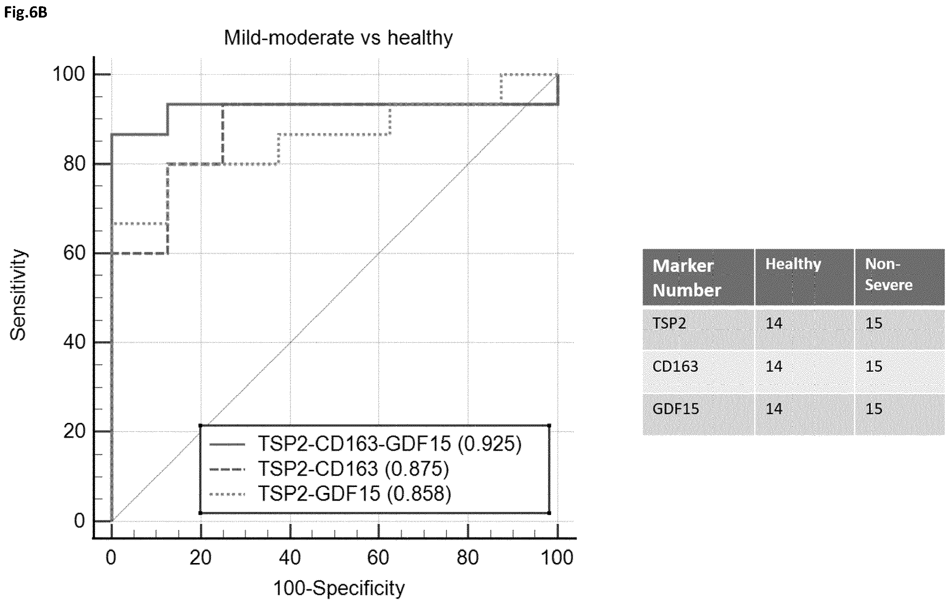

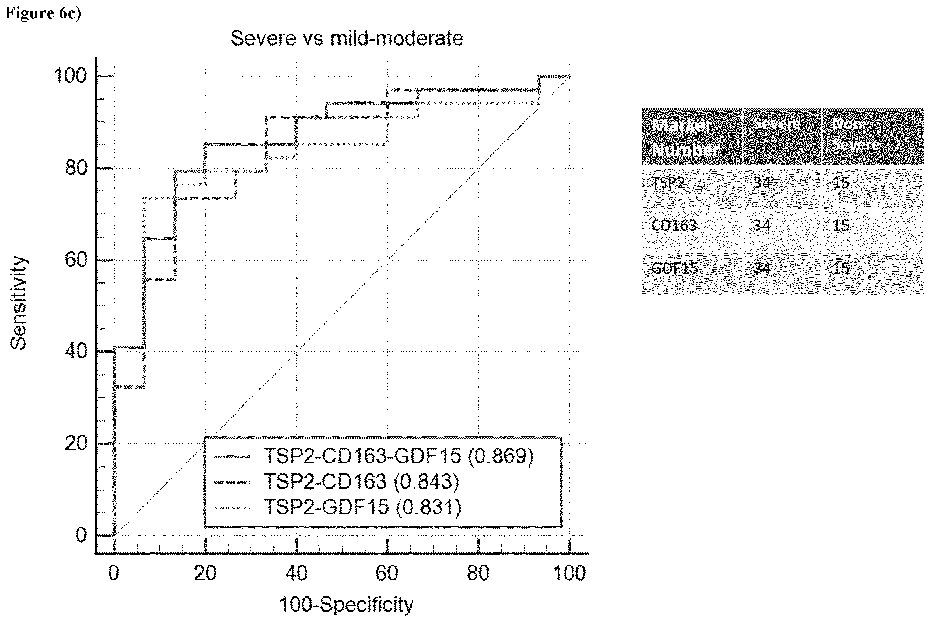

Figure 6: Algorithms of combinations of two of the three serum/plasma-biomarkers TSP2, GDF15 and CD163 according to the invention allow early differentiation (A) severe COVID19 disease from healthy, (B) of mild-moderate COVID19 disease from healthy, and (C) mild-moderate from severe COVID19 within 1-2 days after hospital admission. Predictive values are shown in comparison to the triple biomarker algorithm.

## EXAMPLES

### Summary

In the context of the present invention, the inventors used and optimized commercially available ELISA tests for the markers CD163, GDF15 and IGFBP7 (R&D systems). An ELISA for Tsp-2 based on the inventors own recombinant expression of the protein and two commercial antibodies (mouse monoclonal and goat polyclonal) was established and validated.

Serum/plasma samples from 87 hospitalized patients with well-defined diagnosis of COVID19 and SPD in the acute phase of their diseases and in early follow-up up to 4 weeks were collected. Sub-cohorts of patients who survived with complete remission or long-term sequelae, especially long-COVID19 syndrome were followed for up to 4 months. In those patients who did not survive due to their COVID19 disease or SPD the overall gene expression in lungs of was determined from post-mortem lungs by transcriptome analysis and correlated to the serum/plasma levels of our markers CD163, Tsp2 and GDF15.

### Methods

### Patient selection, samples, and study groups

In the present study, the inventors analyzed pulmonary autopsy specimens from 31 patients who died from respiratory failure caused by SARS-CoV-2 infection (mean age $73\pm11.2$ yrs 22 male victims, 9 female victims, mean hospitalization time $15.3\pm10.3$ days) and compared them with lungs from seven patients who died from pneumonia caused by influenza A virus subtype H1N1 (A[H1N1]) - a strain associated with the 1918 and 2009 influenza pandemics (mean age $57.4\pm9.7$ yrs, 5 male victims, 2 female victims, mean hospitalization time $11.6\pm5.4$ days). The lungs from patients with influenza were archived tissue from the 2009 pandemic and were chosen for the best possible match with respect to age, sex, and disease severity from among the autopsies performed at the Hannover Medical School. 19 lungs that had been donated but not used for transplantation served as uninfected control specimens from the Hannover Medical School and the University of Leuven. The influenza group consisted of lungs from two female and five male patients with mean ages of $62.5\pm4.9$ years and $55.4\pm10.9$ years, respectively. Five of the uninfected lungs were from female donors (mean age, $68.2\pm6.9$ years), and five were from male donors (mean age, $79.2\pm3.3$ years). Fresh lung explants of 18 patients with different subtypes of interstitial lung disease (ILD) were included in this study. All diagnoses were made by experienced pulmonary pathologists following the European Respiratory Society and American Thoracic Society guidelines.

Patient groups consisted of patients with characteristic radiological and histological patterns: UIP (n = 6, 0 female donors, age at transplantation (Tx) $61.4 \pm 6.8$ years [mean $\pm$ SD]), idiopathic NSIP (n = 6, 4 female, age at Tx $52.3 \pm 9.2$ years) as well as AFE (n = 6, 5 female, age at Tx $40.7 \pm 27.8$ years) in the context of chronic graft-versus-host disease, following radio/chemotherapy, with the histological hallmarks of chronic lung allograft dysfunction and idiopathic pulmonary fibrosis (IPF). A total of 118 serum and plasma samples were used from the Hannover Unified Biobank (HUB). 81 samples were from hospitalized COVID-19 patients with follow-ups after convalescence. Plasma samples were obtained from COVID-19 patients classified clinically as mild, moderate, severe, and post-COVID, and compared to 20 samples from hospitalized patients with influenza-related ARDS and from 17 ILD patients (6 IPF, 5 NSIP, and 6 ILD with acute exacerbation).

### RNA extraction and gene expression analysis.

RNA was isolated using the Maxwell® RNA extraction system (Promega, Madison, Wisconsin) and following quality control via Qubit analysis (ThermoFisher, Waltham, Massachusetts) used for further analysis. All mRNA expression data was obtained via the nCounter® Analysis System (NanoString Technologies, Seattle, WA) using the PanCancer Progression Panel (770 genes including 30 reference genes). Normalization of raw counts was performed using the nSolver™ analysis software version 3.0 (NanoString Technologies, Seattle, WA) and a modified version of the nCounter® advanced analysis module (version 1.1.5). The normalization process included positive normalization (geometric mean), negative normalization (arithmetic mean) and reference normalization (geometric mean) using the five most suitable reference genes from the total of 30 available reference genes selected by the geNorm algorithm.

Functional analysis.

In order to gain insight on the regulation of physiological functions conveyed by the mRNA expression in different ILD entities, the inventors made use of the Ingenuity Pathway Analysis tool (IPA, QIAGEN) The log2 gene expression data of each sample relative to the median gene expression in the controls was committed to the software as an individual observation. The subsequent molecule function activity prediction of IPA was executed with the default settings, thus using information from both in vivo and in vitro experiments. Any z-score obtained as a result gives a quantitative estimate of how a biological function is presumably regulated based on the observed differences in gene expression. https://www.qiagenbioinformatics.com/products-/ingenuity-pathway-analysis. The inventors considered p-values significant according to the following levels of confidence: $p < 0.05$ (*), $p < 0.01$ (**) and $p < 0.001$ (***).

**Determination of plasma GDF15, CD163, TSP2 and IGFBP7 by enzyme linked immunosorbent assay (ELISA)**

Plasma GDF15 and CD163, levels were measured according to the manufacturer's instructions using DuoSet ELISAs from R&D systems with altered antibody combinations to increase sensitivity and specificity. Assay linearity allowed reliable measurements between 62.5 to 1000 pg/ml for GDF15 and 325 to 20,000 pg/ml for CD163. Normal plasma levels were confirmed with samples of the inventors' own plasma repository, and inter- and intra- assay variabilities below 15% were reached using 5 normal and 5 pathological plasmas in 6 replicates. Plasma matricellular markers thrombospondin-2 (TSP2) and insulin like growth factor binding protein 7 (IGFBP7) were measured using in-house developed ELISAs. Briefly, 96 well plates were coated with 100 ng/well of monoclonal antibodies from LSBio (LS-C132440) and Thermofischer (MA5-30520). Plates were incubated overnight at 4°C followed by washing with PBS/0.1% Tween-20 and blocking with 1% BSA for 1 hr at RT. After a second wash, samples along with standards (in-house recombinant TSP2 and IGFBP7 and blank controls) were added to the wells, incubated for 2 hrs at RT followed by washing. Goat polyclonal anti-TSP2 and anti-IGFBP7 antibodies purchased from R&D systems (AF1635 and AF1334) were labeled with horseradish peroxidase (HRP) using an HRP conjugation kit (Abcam, ab 102890). Predetermined HRP-antibody dilutions were added to the plates for 1 hr, followed by washing and addition of 100 $\mu$l ready-to-use tetramethyl-benzidine (Thermofisher; Cat: N301) for 15 minutes at RT in the dark. The reaction was stopped with 50 $\mu$l 1% HCL, and OD was measured at 450 nm with 570 nm as reference. The linear range was between 162 to 10,000 pg/ml for TSP2 and 325 to 5,000 pg/ml for IGFBP7. Both assays were highly reliable, and intra- and interassay variabilities were below 10% and 13%, respectively.

**Statistics and calculation of biomarker algorithms**

One-way ANOVA test was performed using the GraphPad Prism 9 and the logistic regression analysis was performed in MedCalc statistical software using backward stepwise ratio model including TSP2, CD163 and GDF15. The models differentiating mild-moderate COVID19 and severe COVID19 from healthy and severe from mild-moderate COVID19 were calculated as follows for the triple combination:

$$\mathrm{logit(p)} = -7.3 + 0.02 \cdot \mathrm{TSP2} + 0.004 \cdot \mathrm{GDF15} + 0.01 \cdot \mathrm{CD163}; \ \mathrm{logit(p)} = -10.65 + (-0.05) \cdot \mathrm{TSP2} + 0.007 \cdot \mathrm{GDF15} + 0.0176 \cdot \mathrm{CD163} \ \& \ \mathrm{logit(p)} = -2.5 + 0.005 \cdot \mathrm{TSP2} + 0.0005 \cdot \mathrm{GDF15} + 0.002 \cdot \mathrm{CD163}.$$

**Results**

Serum/plasma markers CD163, Tsp2 and GDF15 predicted the course of mild-moderate COVID19 vs healthy controls with areas under receiver operating curves (AUROCs) of 0.767-0.875, and severe COVID19 vs mild to moderate COVID19 with AUROCs of 0.692-0.831.

Similar results were obtained for other SPDs, like influenza. The combination of the three biomarkers is a superior predictor of disease as compared to the single biomarker levels, reaching AUROCs of 0.925 and 0.869, respectively.

Patients with long-term follow-up showed trajectories either with lower but continuously elevated biomarker levels, or with biomarker levels that normalized, in correlation to incomplete vs full recovery. The three selected biomarkers furthermore correlated with upregulated expression of the respective genes in lungs of patients with severe COVID19 vs non-COVID lungs.

**Claims**

1.  A method for detecting and/or predicting the severity and/or chronicity of a systemic pulmonary disease (SPD) in a patient, comprising

    determining the expression of at least two markers selected from the group consisting of macrophage marker CD163, matricellular fibrosis marker thrombospondin-2 (Tsp2), and (cardiovascular) inflammation/repair marker growth differentiation factor 15 (GDF-15) in a sample obtained from said patient,
    wherein an increase of the expression of the at least two markers detects and/or predicts the severity and/or chronicity of the systemic pulmonary disease (SPD) in the patient, when compared to a healthy control and/or a mild to moderate SPD control.

2.  The method according to claim 1, wherein the expression of all three markers is determined, and wherein preferably furthermore the expression of the marker IGFBP7 is determined.

3.  The method according to claim 1 or 2, wherein said SPD is selected from the group consisting of influenza infection, pulmonary fibrosis, idiopathic pulmonary fibrosis, interstitial lung disease (ILD), acute respiratory distress syndrome (ARDS), influenza A virus infection, and coronavirus infection, such as, for example, COVID19 and long COVID19.

4.  The method according to any one of claims 1 to 3, wherein the detecting and/or predicting the severity and/or chronicity comprises at least one of i) distinguishing severe COVID19 disease or ARDS from milder SPD, and/or from moderate to mild COVID19 disease, and ii) predicting severe COVID19 disease or ARDS from milder SPD, and/or from moderate to mild COVID19 disease in the patient.

5.  The method according to any one of claims 1 to 3, wherein the detecting and/or predicting the severity and/or chronicity comprises at least one of i) differentiation of mild-moderate COVID19 disease from healthy controls within the first two days of admission of the patient, and ii) differentiating an emerging severe from a mild-moderate COVID19 disease within the first two days of admission of the patient.

6.  The method according to claim 1 or 3, wherein the detecting and/or predicting the severity and/or chronicity comprises differentiating a severe vs mild evolution of long-COVID syndrome in said patient based on an increase of the expression of the markers CD163 and TSP2, such as, for example, a re-spiking of the markers in the patient.

7.  A method for diagnosing the severity and/or chronicity of a systemic pulmonary disease (SPD) in a patient, comprising performing the method according to any one of claims 1 to 6, and further comprising the step of diagnosing the severity and/or chronicity of the systemic pulmonary disease (SPD) in the patient, wherein an increase of the expression of the at least two markers is diagnostic for a higher severity and/or longer chronicity of the systemic pulmonary disease (SPD) in the patient, when compared to a healthy control, an earlier sample form the same patient and/or a control sample.

8.  The method according to any one of claims 1 to 7, wherein the sample is obtained from a patient that is undergoing or has received treatment against an SPD.

9.  A method for monitoring the severity and/or chronicity of a systemic pulmonary disease (SPD) in a patient, comprising performing the method according to claim 7 or 8, and further comprising the step of monitoring the severity and/or chronicity of the systemic pulmonary disease (SPD) in the patient based on a comparison with at least one determination as obtained when performing the method according to claim 7 or 8 on an earlier sample form the same patient and/or on a control sample.

10. The method according to any one of claims 7 to 9, wherein an increased level of expression is indicative of a more severe disease progression.

11. A method for predicting the efficacy of a treatment of a systemic pulmonary disease (SPD) in a patient, comprising performing the method according to claim 8, and further comprising the step of predicting the efficacy of the treatment, wherein an decrease of the expression of the at least two markers is predictive for an efficient treatment of the systemic pulmonary disease (SPD) in the patient, when compared to a healthy control, an earlier sample form the same patient and/or a control sample.

12. A method for identifying a compound for the prevention or treatment of a systemic pulmonary disease (SPD) in a subject, comprising the steps of administering at least one candidate compound to said subject, and determining the expression of at least two markers selected from the group consisting of macrophage marker CD163, matricellular fibrosis marker thrombospondin-2 (Tsp2), and (cardiovascular) inflammation/repair marker growth differentiation factor 15 (GDF-15) in a sample obtained from said subject in the presence of said at least one candidate compound and comparing it to the expression of said markers in a sample obtained from said subject in the absence of said at least one candidate compound and/or in a control sample, wherein an decrease of the expression of the at least two markers identifies a compound for the prevention or treatment of a systemic pulmonary disease (SPD) in the subject, wherein preferably the expression of all three markers is determined, and wherein preferably furthermore the expression of the marker IGFBP7 is determined.

13. A pharmaceutical composition comprising at least one compound for the prevention or treatment of a systemic pulmonary disease (SPD) as identified according to any one of claim 12, together with at least one pharmaceutically acceptable carrier or diluent.

14. The at least one compound for the prevention or treatment of a systemic pulmonary disease (SPD) as identified according to claim 12 or the pharmaceutical composition according to claim 13 for use in medicine, preferably for use in the prevention or treatment of a systemic pulmonary disease (SPD), such as influenza infection, such as, for example, pulmonary fibrosis, idiopathic pulmonary fibrosis, interstitial lung disease (ILD), acute respiratory distress syndrome (ARDS), influenza A virus infection), and coronavirus infection, such as, for example, COVID19 and long COVID19 in a patient, wherein preferably the patient is undergoing or has received additional treatment against the SPD.

15. A kit, such as, for example, a diagnostic or therapeutic kit, comprising materials for performing the method for detecting and/or predicting the severity and/or chronicity of a systemic pulmonary disease (SPD) in a patient according to any one of claims 1 to 6 and 8, the method for diagnosing the severity and/or chronicity of a systemic pulmonary disease (SPD) in a patient according to any one of claims 7 or 8, the method for monitoring the severity and/or chronicity of a systemic pulmonary disease (SPD) in a patient according to claim 9 or 10, the method for predicting the efficacy of a treatment of a systemic pulmonary disease (SPD) in a patient according to claim 11, or the method for identifying a compound for the prevention or treatment of a systemic pulmonary disease (SPD) in a subject according to claim 12.

**Fig.1**

EP 4 336 186 A1

Fig.2

## TSP2

## GDF15

## IGFBP7

## CD163

EP 4 336 186 A1

Figure 3

**Fig.3A**

Figure 3 (continued)

Fig.3B

Fig.4

## Mild-moderate vs healthy

| Number Marker | Healthy | Non-Severe |
|---|---|---|
| TSP2 | 14 | 15 |
| CD163 | 14 | 15 |
| GDF15 | 14 | 15 |

Legend:
- CD163 (0.767)
- TSP2 (0.804)
- GDF15 (0.875)
- CD163|TSP2|GDF15 (0.925)

Sensitivity vs 100-Specificity

EP 4 336 186 A1

Fig.5

## Severe vs mild-moderate

| Marker Number | Severe | Non-Severe |
|---|---|---|
| TSP2 | 34 | 15 |
| CD163 | 34 | 15 |
| GDF15 | 34 | 15 |

CD163 (0.692)
TSP2 (0.799)
GDF15 (0.831)
CD163-TSP2-GDF15 (0.869)

EP 4 336 186 A1

Fig.6A

Severe vs healthy

| Marker Number | Severe | Healthy |
|---|---|---|
| TSP2 | 34 | 14 |
| CD163 | 34 | 14 |
| GDF15 | 34 | 14 |

Fig.6B

## Mild-moderate vs healthy

| Marker Number | Healthy | Non-Severe |
|---|---|---|
| TSP2 | 14 | 15 |
| CD163 | 14 | 15 |
| GDF15 | 14 | 15 |

**Figure 6c)**

Severe vs mild-moderate

Legend:
- TSP2-CD163-GDF15 (0.869)
- TSP2-CD163 (0.843)
- TSP2-GDF15 (0.831)

Axes: Sensitivity (y-axis), 100-Specificity (x-axis)

| Marker Number | Severe | Non-Severe |
|---|---|---|
| TSP2 | 34 | 15 |
| CD163 | 34 | 15 |
| GDF15 | 34 | 15 |

Europäisches
Patentamt

European
Patent Office

Office européen
des brevets

## EUROPEAN SEARCH REPORT

Application Number

EP 22 19 5149

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/104212 A1 (AMGEN INC [US]) 19 May 2022 (2022-05-19) | 15 | INV. G01N33/68 G01N33/569 |
| Y | * whole document, in particular claim 1, 2, 4, 16, 20, 26, 37, 45, 57, 65, 83; par. 32, 44-48, 205, 207, 208, 266-270; table 1 * | 1-15 | |
| Y | WO 2019/094777 A1 (GILEAD SCIENCES INC [US]) 16 May 2019 (2019-05-16) * whole document, in particular par. 49 147-153; claim 1, 7, 29 * | 1-5, 7-15 | |
| Y | WO 2022/103971 A1 (WISTAR INST [US]) 19 May 2022 (2022-05-19) * the whole document, in particular claim 1; ; p. 5, 1. 30 – p. 6, 1. 9; p. 22, 1. 23-25; p. 27, 1. 21-31; p. 38, 1. 26 – p. 39, 1. 9; p. 42, 1. 2-5; fig. 2 * | 1-15 | |
| A | WO 2012/122228 A2 (UNIV TEMPLE [US]; MERALI SALIM [US] ET AL.) 13 September 2012 (2012-09-13) * the whole document * | 1-15 | TECHNICAL FIELDS SEARCHED (IPC) G01N |
| A | WO 2022/114984 A1 (QATAR FOUND EDUCATION SCIENCE & COMMUNITY DEV [QA] ET AL.) 2 June 2022 (2022-06-02) * the whole document * | 1-15 | |
| A | WO 2019/099706 A1 (BETH ISRAEL DEACONESS MEDICAL CT INC [US]; UNIV NEBRASKA [US]) 23 May 2019 (2019-05-23) * the whole document * | 1-15 | |

-/--

The present search report has been drawn up for all claims

| Place of search | Date of completion of the search | Examiner |
|---|---|---|
| The Hague | 9 February 2023 | Chrétien, Eva Maria |

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JONIGK DANNY ET AL: "Organ manifestations of COVID-19: what have we learned so far (not only) from autopsies?", VIRCHOWS ARCHIV, SPRINGER BERLIN HEIDELBERG, BERLIN/HEIDELBERG, vol. 481, no. 2, 1 April 2022 (2022-04-01) , pages 139-159, XP037920443, ISSN: 0945-6317, DOI: 10.1007/S00428-022-03319-2 [retrieved on 2022-04-01] * the whole document * | 1-15 | |
| A | WO 2022/017980 A1 (HOFFMANN LA ROCHE [CH]; ROCHE DIAGNOSTICS GMBH [DE] ET AL.) 27 January 2022 (2022-01-27) * the whole document * ----- | 1-15 | |

TECHNICAL FIELDS
SEARCHED      (IPC)

The present search report has been drawn up for all claims

| Place of search | Date of completion of the search | Examiner |
|---|---|---|
| The Hague | 9 February 2023 | Chrétien, Eva Maria |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.........................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 19 5149

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2023

| Patent document cited in search report | | Publication date | Patent family member(s) | | Publication date |
|---|---|---|---|---|---|
| WO 2022104212 | A1 | 19-05-2022 | NONE | | |
| WO 2019094777 | A1 | 16-05-2019 | EP | 3710599 A1 | 23-09-2020 |
| | | | US | 2020340060 A1 | 29-10-2020 |
| | | | WO | 2019094777 A1 | 16-05-2019 |
| WO 2022103971 | A1 | 19-05-2022 | NONE | | |
| WO 2012122228 | A2 | 13-09-2012 | US | 2014030735 A1 | 30-01-2014 |
| | | | US | 2016213700 A1 | 28-07-2016 |
| | | | WO | 2012122228 A2 | 13-09-2012 |
| WO 2022114984 | A1 | 02-06-2022 | NONE | | |
| WO 2019099706 | A1 | 23-05-2019 | CA | 3082591 A1 | 23-05-2019 |
| | | | EP | 3710831 A1 | 23-09-2020 |
| | | | WO | 2019099706 A1 | 23-05-2019 |
| WO 2022017980 | A1 | 27-01-2022 | EP | 3943946 A1 | 26-01-2022 |
| | | | WO | 2022017980 A1 | 27-01-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2022017980 A

### Non-patent literature cited in the description

- **MANDAL S et al.** ARC Study Group. 'Long-COVID': a cross-sectional study of persisting symptoms, biomarker and imaging abnormalities following hospitalisation for COVID-19. *Thorax,* 10 November 2020, vol. 76 (4), 396-398
- **AVOUAC J ; CAUVET A ; STEELANDT A ; SHIRAI Y ; ELHAI M ; KUWANA M et al.** Improving risk-stratification of rheumatoid arthritis patients for interstitial lung disease. *PLoS ONE,* 2020, vol. 15 (5), e0232978, https://doi.org/10.1371/journal.pone.0232978
- **AHMED DS et al.** Coping With Stress: The Mitokine GDF-15 as a Biomarker of COVID-19 Severity. *Front Immunol.,* 16 February 2022, vol. 13, 820350, https://doi.org/10.3389/fimmu.2022.820350
- **MYHRE PL et al.** Growth Differentiation Factor 15 Provides Prognostic Information Superior to Established Cardiovascular and Inflammatory Biomarkers in Unselected Patients Hospitalized With COVID-19. *Circulation,* 15 October 2020, vol. 142 (22), 2128-2137
- **GOMEZ-RIAL J et al.** Increased Serum Levels of sCD14 and sCD163 Indicate a Preponderant Role for Monocytes in COVID-19 Immunopathology. *Front Immunol.,* 23 September 2020, vol. 11, 560381
- Remington's Pharmaceutical Sciences. Mack Publishing Co, 1991
- **BAUER et al.** Pharmazeutische Technologic. Govi-Verlag, 1997